# EUROPÄISCHE PATENTSCHRIFT

(11) **EP 0 909 749 B1**
(45) Veröffentlichungstag und Bekanntmachung des Hinweises auf die Patenterteilung: **28.08.2002**
(21) Anmeldenummer: 98117120.0
(22) Anmeldetag: 10.09.1998
(51) Int. Cl.: C07C 29/36, C07C 29/58

(54) **Verfahren zur Herstellung von Pentafluorpentanol**
Process for the preparation of pentafluoropentanol
Procédé pour la préparation de pentafluoropentanol

(30) Priorität: 14.10.1997 DE 19745374; 05.11.1997 DE 19748775
(43) Veröffentlichungstag der Anmeldung: 21.04.1999
(73) Patentinhaber: BAYER AG, 51368 Leverkusen (DE)
(72) Erfinder: Rock, Michael-Harold Dr., 2500 Valby (DK); Marhold, Albrecht Dr., 51373 Leverkusen (DE)

(56) Entgegenhaltungen:
- WO-A-93/13123
- X. LI, ET AL.: "Laboratory scale preparation of 4,4,5,5,5-pentafluoropentane-1- thiol: an important chain of anti-breast cancer agents" TETRAHEDRON LETTERS, Bd. 35, Nr. 49, 5. Dezember 1994, Seiten 9141-9144, XP002090321 OXFORD, GB
- J.D. PARK, ET AL.: "Free-radical catalysed addition of unsaturated alcohols to perhaloalkanes" JOURNAL OF ORGANIC CHEMISTRY, Bd. 26, Nr. 6, 13. Juni 1961, Seiten 2089-2095, XP002090322 WASHINGTON, DC, US
- U. LARSSON, ET AL.: "Synthesis of amino acids with modified principal properties 1. Amino acids with fluorinated side chains" ACTA CHEMICA SCANDINAVICA, Bd. 47, Nr. 4, April 1993, Seiten 380-390, XP002090323 AMSTERDAM, NL

## Beschreibung

Die vorliegende Erfindung betrifft ein verbessertes Verfahren zur Herstellung von 4,4,5,5,5-Pentafluor-1-pentanol aus Perfluorethyliodid.

4,4,5,5,5-Pentafluor-1-pentanol ist ein wertvolles Zwischenprodukt für die Herstellung von Pharmazeutika (siehe z.B. DE-A 41 32 182 und Tetrahedron Lett. 35, 9141 (1994)).

Aus der Literatur sind mehrere Verfahren zur Herstellung von 4,4,5,5,5-Pentafluor-1-pentanol bekannt.

Zwei davon gehen von Perfluorethyliodid und Propargylalkohol aus. Nach dem einen (Tetrahedron Lett. 35, 9141 (1994)) führt die mit Natriumdithionit/Natriumbicarbonat initiierte radikalische Addition in guter Ausbeute zum 4,4,5,5,5-Pentafluor-2-iod-2-penten-1-ol. Im zweiten Schritt wird dieses dann wiederum in guter Ausbeute in Gegenwart von zwei Äquivalenten Triethylamin und Platinoxid katalytisch mit Wasserstoff zugleich hydriert und dehalogeniert. Von entscheidendem Nachteil dieses Verfahrens ist jedoch die benötigte Menge des teuren Platinoxids (2,5 g für weniger als 30 g Produkt).

Nach einem anderen Verfahren (DE-A 41 32 182) wird die ultraschallbeschleunigte Addition über das in Gegenwart von Zink und Kupfer(I)-iodid erzeugte Perfluoralkylcuprat zum 4,4,5,5,5-Pentafluor-2-penten-1-ol, wie in J. Am. Chem. Soc. 107, 5186 (1985) beschrieben, durchgeführt. Auf diese folgt eine katalytische Hydrierung mit Platinoxid als Katalysator. Während man hier mit 5 % der Katalysatormenge gegenüber der o.a. enthalogenierenden Hydrierung auskommt, ist hier die schlechte Ausbeute im ersten Reaktionsschritt von nur 46 % d. Th. und der technisch aufwendige Gebrauch von Ultraschall nachteilig.

Weiterhin ist bei beiden Verfahren ein Gefahrenpotential durch die Acetylenbindung im Propargylalkohol, insbesondere bei den lokal auftretenden hohen Temperaturen bei Ultraschallbestrahlung, zu berücksichtigen.

Die radikalische Addition von Pentafluorethyliodid an Allylalkohol liefert zunächst 4,4,5,5,5-Pentafluor-2-iod-pentanol (siehe zum Beispiel WO 93/13123). Dieses kann gemäß N.O. Brace (J. Fluorine Chem., 20, 313-327 (1983)) zum Beispiel mit Wasserstoff und Raney-Nickel als Katalysator in Gegenwart von Kaliumacetat enthalogeniert werden. Allerdings tritt in diesem Fall als Nebenprodukt das unerwünschte Isomer 4,4,5,5,5-Pentafluorpentan-2-ol auf, das die Folge einer intermediären Bildung eines 1,2-Epoxids ist. In WO 93/13123 wird daher als Alternative dazu die Hydrierung an Palladium- oder Raney-Nickel-Katalysatoren ohne Basenzusatz erwähnt.

Ein weiteres Verfahren ist dreistufig und geht ebenfalls von Perfluorethyliodid aus (Acta Chem. Scand. 47, 380 (1993)). Eine durch 2,2-Azo-bis-isobuttersäurenitril (AIBN) initiierte radikalische Addition an Allylacetat ergibt 5-Acetoxy-4-iod-1,1,1,2,2-pentafluorpentan, welches radikalisch mit Tributylzinnhydrid dehalogeniert wird. Das erhaltene 5-Acetoxy-1,1,1,2,2-pentafluorpentan wird im abschließenden Schritt mit Lauge verseift. Von Nachteil ist bei diesem Verfahren die höhere Anzahl von Reaktionsstufen und die schwer kontrollierbare und damit im größeren Maßstab praktisch nicht durchführbare Reaktion mit Tributylzinnhydrid.

Es ist schließlich auch noch bekannt, daß man Pentafluorethyliodid mit Allylalkohol in Gegenwart von Benzoylperoxid umsetzen kann (J. Org. Chem. 26, 2089 (1961)). Die Ausbeute beträgt selbst nach 14 Tagen Reaktionszeit nur 55 % d.Th.

Im Handel ist nur 4,4,5,5,5-Pentafluor-1-pentanol erhältlich, das eine Reinheit von ca. 95 % aufweist. Zur Herstellung von Wirkstoffen ist eine derartige Reinheit nicht ausreichend.

Es wurde nun ein Verfahren zur Herstellung von 4,4,5,5,5-Pentafluor-1-pentanol aus Perfluorethyliodid gefunden, das dadurch gekennzeichnet ist, daß man zunächst Perfluorethyliodid in Gegenwart von Radikalinitiatoren, ausgewählt aus der Gruppe Dithionit/Bicarbonat-Gemische und komplexe Metallverbindungen, die ein oder mehrere Zentralatome aus der Gruppe Eisen, Cobalt und Nickel und Dienyl- und/oder Carbonylliganden enthalten, an Allylalkohol addiert und dann das so erhaltene 4,4,5,5,5-Pentafluor-2-iod-1-pentanol in Gegenwart eines Katalysators, eines Säurebindemittels, das ausgewählt ist aus der Gruppe Hydrogencarbonate, primäre, sekundäre oder tertiäre Amine oder Alkanolamine, und eines Verdünnungsmittels hydrogenolytisch dehalogeniert.

Die als Ausgangsmaterialien für das erfindungsgemäße Verfahren benötigten Chemikalien Perfluorethyliodid und Allylalkohol sind kommerziell erhältlich.

Bevorzugt als Radikalinitiator ist das System Natriumdithionit/Natriumbicarbonat und die dimere Metallverbindung Cyclopentadienyleisendicarbonyl [Cp(CO)₂Fe]₂.

Dithionit/Bicarbonat-Gemische können pro Mol Dithionit z.B. 0,5 bis 2 mol Bicarbonat enthalten. Vorzugsweise liegt dieses Verhältnis bei 1:0,7 bis 1,5. Beispielsweise kann man soviel Dithionit/Bicarbonat-Gemisch oder komplexe Metallverbindungen einsetzen, daß pro Mol Perfluorethyliodid 1 bis 5 mol, vorzugsweise 1,1 bis 3 mol, Dithionit zum Einsatz gelangen.

Die Radikalinitiatoren kann man auch in geringeren Mengen einsetzen, beispielsweise 0,01 bis 0,5 mol, insbesondere 0,05 bis 0,2 mol, pro Mol Perfluorethyliodid.

Die Addition kann in Gegenwart oder Abwesenheit eines Verdünnungsmittels durchgeführt werden. Beim Einsatz von Dithionit/Bicarbonat-Gemischen ist es vorteilhaft, Wasser oder Gemische von wasserlöslichen organischen Lösungsmitteln mit Wasser (Wassergehalt vorzugsweise mindestens 30 Vol.-%) einzusetzen. Beim Einsatz von organischen, von Acylgruppen freien Radikalinitiatoren, von Licht oder von komplexen Metallverbindungen kann man bevorzugt organische, z.B. mit Wasser unmischbare oder mischbare Lösungsmittel einsetzen oder auch ohne Verdünnungsmittel arbeiten. Als mit Wasser mischbare Lösungsmittel kommen z.B. in Frage: Nitrile wie Acetonitril, Propionitril, n- und i-Butyronitril und Alkohole wie Methanol, Ethanol, n- und i-Propanol, n-, i-, s- und t-Butanol, Ethandiol, Propan-1,2-diol, Ethoxyethanol, Methoxyethanol, Diethylenglykolmonomethylester und Diethylenglykolmonoethylester. Bevorzugt sind Acetonitril/Wasser-Gemische. Pro Mol Pentafluorethyliodid kann man z.B. 50 ml bis 3 l Verdünnungsmittel einsetzen.

Bei der Addition kann die Temperatur in einem weiten Bereich variiert werden. Bevorzugt arbeitet man beim Einsatz von Dithionit/Bicarbonat-Gemischen bei -50 bis +25°C, besonders bevorzugt bei -20 bis +25°C. Dabei arbeitet man im allgemeinen unter Normaldruck. Es ist auch möglich unter vermindertem oder erhöhtem Druck zu arbeiten. Beim Einsatz anderer Radikalinitiatoren können gegebenenfalls andere Temperaturen gewählt werden. Es ist aber in jedem Fall darauf zu achten, daß sich aus dem Radikalinitiator genügend Radikale bilden.

Man kann Pentafluorethyliodid und Allylalkohol beispielsweise in molaren Verhältnissen von 1:0,7 bis 20 einsetzen. Bevorzugt beträgt dieses Verhältnis 1:0,9 bis 2.

Nach Beendigung der Addition kann man das Reaktionsgemisch beispielsweise durch Extraktion mit einem geeigneten Lösungsmittel aufarbeiten. Geeignete Lösungsmittel hierfur sind beispielsweise Ethylacetat und Methyl-tert.-butylether. Man kann dem Reaktionsgemisch auch Wasser hinzufügen und bei vermindertem Druck leichtflüchtige Bestandteile abziehen.

Als Katalysator für den zweiten Reaktionsschritt, die hydrogenolytische Dehalogenierung, kann man beispielsweise heterogene Hydrierkatalysatoren wie Platinoxid, Palladium-auf-Kohle oder Raney-Nickel verwenden. Vorzugsweise wird Palladium-auf-Kohle mit einem Gehalt von 2 bis 10 Gew.-% Palladium eingesetzt. Die Katalysatormenge kann in weiten Grenzen variiert werden. Beispielsweise kann man 0,001 bis 1 Gew.-%, vorzugsweise 0,01 bis 0,1 Gew.-% Katalysator (gerechnet als Metall) bezogen auf 4,4,5,5,5-Pentafluor-2-iod-1-pentanol verwenden.

Als Säurebindemittel für die hydrogenolytische Dehalogenierung werden Hydrogencarbonate wie Natrium- und Kaliumhydrogencarbonat, primäre, sekundäre und tert. Amine wie Methylamin, Dimethylamin, Ethylamin, Diethylamin, Trimethylamin, Triethylamin, Tributylamin, N,N-Dimethylanilin, N,N-Dimethylbenzylamin, Pyridin, N-Methylpyridin, N-Methylmorpholin, N,N-Dimethylaminopyridin, Diazabicyclooctan (DABCO), Diazabicyclononen (DBN) und Diazabicycloundecen (DBU) und Alkanolamine wie Methylolamin, Dimethylolamin, Trimethylolamin, Ethanolamin, Diethanolamin, Triethanolamin und die entsprechenden C₃-C₆-Alkanolamine eingesetzt. Vorzugsweise wird Triethylamin oder Monoalkanolamin eingesetzt.

Als Verdünnungsmittel zur Durchführung der erfindungsgemäßen hydrogenolytischen Dehalogenierung kommen z.B. Wasser, organische Lösungsmittel und beliebige Mischungen davon in Betracht. Als organische Lösungsmittel seien beispielhaft genannt: aliphatische und alicyclische Kohlenwasserstoffe wie Petrolether, Hexan, Heptan, Cyclohexan und Methylcyclohexan, Ether wie Diethyl-, Diisopropyl-, Methyl-t-butyl- und Methyl-t-amylether, Dioxan, Tetrahydrofuran, 1,2-Dimethoxyethan, 1,2-Diethoxyethan, Diethylenglykoldimethylether und Anisol, Ester wie Methyl-, Ethyl- und Butylacetat und Alkohole wie Methanol, Ethanol, n- und i-Propanol, n-, i-, s- und t-Butanol, Ethandiol, Propan-1,2-diol, Ethoxyethanol, Methoxyethanol, Diethylenglykolmonomethylether und Diethylenglykolmonoethylether. Bevorzugt sind Methyl-t-butylether und Ethylacetat sowie die Kombination von Wasser mit Alkanolaminen als Säurebindemittel.

Bei der hydrogenolytischen Dehalogenierung kann die Reaktionstemperatur in einem weiten Bereich variiert werden. Man kann beispielsweise bei -20 bis +100°C, vorzugsweise bei 0 bis 50°C, arbeiten. Der Wasserstoffdruck kann beispielsweise 5 bis 300 bar, vorzugsweise 20 bis 100 bar, betragen. Beim Einsatz besonders aktiver Katalysatoren ist es auch möglich, im Druckbereich 1 bis 5 bar zu arbeiten. Während der Reaktion ist der gewünschte Wasserstoffdruck gegebenenfalls durch Nachdosierung aufrecht zu erhalten.

Pro Mol 4,4,5,5,5-Pentafluor-2-iod-1-pentanol kann man z.B. 0,9 bis 2 Äquivalente, vorzugsweise 1 bis 1,5 Äquivalente Säurebindemittel und 100 bis 1 000 ml Verdünnungsmittel einsetzen.

Es ist ein wesentliches Merkmal des erfindungsgemäßen Verfahrens, daß man das 4,4,5,5,5-Pentafluor-2-iod-1-pentanol in Gegenwart eines Verdünnungsmittels, eines Katalysators und eines Säurebindemittels hydriert. Beispielsweise kann man dazu den Katalysator, das Säurebindemittel und das Verdünnungsmittel unter einer Wasserstoffatmosphäre vorlegen, und das 4,4,5,5,5-Pentafluor-2-iod-1-pentanol sowie gegebenenfalls Wasserstoff im Maße des Verbrauches unter Aufrechterhaltung des Druckes hinzudosieren. Man kann auch das Edukt, den Katalysator, das Verdünnungsmittel und das Säurebindemittel vorlegen und dann durch Aufdrücken von Wasserstoff hydrieren. Man kann 4,4,5,5,5-Pentafluor-l-pentanol in hohen Ausbeuten erhalten.

Die Aufarbeitung kann beispielsweise erfolgen durch Zugabe von Wasser zum Lösen des entstandenen salzförmigen Iodids und anschließende Extraktion des Produktes, gegebenenfalls nach Abfiltration des Hydrierkatalysators. Gegebenenfalls kann man zur weiteren Reinigung die Rohproduktlösung fraktioniert destillieren.

Das erfindungsgemäße Verfahren hat eine Reihe von überraschenden Vorteilen. So benötigt es Hydrierkatalysatoren in üblichen und nicht unverhältnismäßig großen Mengen, es liefert das gewünschte Produkt in hohen Ausbeuten auf technisch einfache Weise, es erfordert nicht die Handhabung von Chemikalien, die wegen ihres Gefahrenpotentials besonderen Aufwand erfordern, es vermeidet die Anwendung von Ultraschall und es ist ohne Probleme auch in größerem Maßstab durchführbar. Es ist vor allem überraschend, daß die erfindungsgemäße Anlagerung von Pentafluorethyliodid an Allylalkohol in Ausbeuten von über 75 % gelingt. Außerdem kann das 4,4,5,5,5-Pentafluor-1-pentanol in höheren, für die Weiterverarbeitung zu Wirkstoffen geeigneten Reinheiten von bis zu 99,9 % erhalten werden. Damit ist 4,4,5,5,5-Pentafluor-1-pentanol wesentlich günstiger zugänglich als bisher.

### Beispiele

### Beispiel 1 Herstellung von 4,4,5,5,5-Pentafluor-2-iod-1-pentanol

440 g Pentafluorethyliodid wurden unter Rühren bei -10 bis -5°C zu einer Lösung von 105 g Allylalkohol, 1 800 ml Acetonitril und 1 400 ml Wasser gegeben. Dann wurde eine Mischung aus 335 g 95 gew.-%igen Natriumdithionit und 170 g Natriumhydrogencarbonat auf einmal zugegeben und die Reaktionsmischung unter Rühren auf einer Temperatur von unter 0°C gehalten. Nach 60 Minuten wurde langsam auf Raumtemperatur erwärmen gelassen. Die erhaltene Suspension wurde in 2 000 ml Wasser gegossen und zweimal mit je 750 ml Ethylacetat extrahiert. Die vereinigten organischen Phasen wurden getrocknet und eingedampft, wobei 430 g (= 80 % d.Th.) 4,4,5,5,5-Pentafluor-2-iod-1-pentanol in Form eines braunen Öles erhalten wurden. Gemäß GC-Untersuchung hatte das Produkt eine 99 %ige Reinheit und wurde ohne weitere Reinigung in Beispiel 4 eingesetzt.

### Beispiel 2

Analog zu Beispiel 1 wurden 130 g Perfluorethyliodid mit 300 g Allylalkohol in 380 ml Wasser durch 100 g Natriumdithionit und 50 g Natriumhydrogencarbonat initiiert umgesetzt. Es wurden 97 g (= 61 % d. Th.) 4,4,5,5,5-Pentafluor-2-iod-1-pentanol von 97 %iger Reinheit (GC) erhalten.

### Beispiel 3

200 ml Methanol wurden mit 240 g Allylalkohol vermischt, auf 0 bis 5°C gekühlt vorgelegt und 1 000 g Pentafluorethyliodid zugegeben. Anschließend erfolgte die Zugabe von 18,0 g dimerem Cyclopentadienyleisendicarbonyl. Das Reaktionsgemisch wurde auf Raumtemperatur gebracht und bei 23 bis 26°C 12 Stunden gerührt. Ein bei -5°C betriebener Rückflußkühler verhinderte das Abdampfen von Pentafluorethyliodid. Zur Aufarbeitung wurden 80 g Wasser in das Reaktionsgemisch gegeben und dann bei 5 mbar die leicht flüchtigen Komponenten abgezogen. Es hinterblieben 1120 g 4,4,5,5,5-Pentafluor-2-iod-1-pentanol. Reinheit 95 % (GC).

### Beispiel 4 Herstellung von 4,4,5,5,5-Pentafluor-1-pentanol

In einem 401-Rührautoklaven wurden 15 l Ethylacetat, 4,5 l Triethylamin und 100 g Palladium-auf-Kohle (5 Gew.-% Palladium) vorgelegt und 60 bar Wasserstoff aufgedrückt. Im Verlaufe von 24 Stunden wurden 8,5 kg 4,4,5,5,5-Pentafluor-2-iod-1-pentanol (erhalten nach der Vorschrift des Beispiels 1) zudosiert und dabei der Wasserstoffdruck auf 40 bis 60 bar gehalten. Nach Beendigung der Zugabe wurde noch 2 Stunden gerührt. Die Reaktion war danach gemäß GC-Analyse vollständig. Nach dem Entspannen wurden 10 l Wasser zur Reaktionsmischung gegeben und der Katalysator durch Filtration abgetrennt. Das Filtrat wurde mit Ethylacetat extrahiert und die vereinigten organischen Phasen getrocknet. Ein Teil des Lösungsmittels wurde zunächst unter Druckerniedrigung bis auf 120 mbar abgedampft. Die verbleibende Mischung wurde im Vakuum destilliert. So wurden 4 400 g (= 88 % d.Th.) 4,4,5,5,5-Pentafluor-1-pentanol in einer Reinheit von 97 % (GC) erhalten. Der Siedepunkt des Produktes betrug 134-135°C bei Normaldruck.

### Beispiel 5

Analog Beispiel 4 wurden 1000 ml Methyltert.-butylether, 270 ml Triethylamin und 10,0 g Palladium-auf-Kohle vorgelegt und 500 g 4,4,5,5,5-Pentafluor-2-iod-1-pentanol mit Wasserstoff enthalogeniert. Es wurden 217 g (= 75 % d. Th.) 4,4,5,5,5-Pentafluor-1-pentanol in einer Reinheit von 98 % (GC) erhalten.

### Beispiel 6

Eine Mischung aus 180 ml Ethanolamin, 820 ml Wasser und 12 g Palladium-auf-Kohle (5 Gew.-% Palladium) wurde vorgelegt und 60 bar Wasserstoff aufgedrückt. Im Verlauf von 12 Stunden wurden 600 g 4,4,5,5,5-Pentafluor-2-iod-1-pentanol (erhalten nach der Vorschrift des Beispiels 1) zudosiert. Die Temperatur wurde auf 30°C und der Wasserstoffdruck bei 50 bis 60 bar gehalten. Nach Beendigung der Zudosierung wurde das Reaktionsgemisch noch 2 Stunden nachgerührt und der vollständige Umsatz gaschromatographisch kontrolliert. Unter reduziertem Druck wurde 4,4,5,5,5-Pentafluor-1-pentanol zusammen mit Wasser aus dem Reaktionsgemisch abdestilliert. Im Destillat bildeten sich zwei Phasen aus. Die organische Phase wurde abgetrennt, die wäßrige Phase mit Dichlormethan extrahiert und das Extrakt mit der organischen Phase vereinigt. Die vereinigten Phasen wurden getrocknet, das Lösungsmittel abdestilliert und das Produkt durch Destillation gereinigt. Es wurden 237 g (= 88 % der Theorie) 4,4,5,5,5-Pentafluor-1-pentanol mit einem Siedepunkt von 130°C bei 1013 mbar erhalten. Reinheit 99,9 % (GC).

## Patentansprüche

1. Verfahren zur Herstellung von 4,4,5,5,5-Pentafluor-1-pentanol aus Perfluorethyliodid, **dadurch gekennzeichnet, daß** man zunächst Perfluorethyliodid in Gegenwart von Radikalinitiatoren, ausgewählt aus der Gruppe Dithionit/Bicarbonat-Gemische und komplexe Metallverbindungen, die ein oder mehrere Zentralatome aus der Gruppe Eisen, Cobalt und Nickel und Dienyl- und/oder Carbonylliganden enthalten, an Allylalkohol addiert und dann das so erhaltene 4,4,5,5,5-Pentafluor-2-iod-1-pentanol in Gegenwart eines Katalysators, eines Säurebindemittels, das ausgewählt ist aus der Gruppe Hydrogencarbonate, primäre, sekundäre oder tertiäre Amine oder Alkanolamine, und eines Verdünnungsmittels hydrogenolytisch dehalogeniert.

2. Verfahren nach Anspruch 1, **dadurch gekennzeichnet, daß** man Dithionit/Bicarbonat-Gemische einsetzt, die pro Mol Dithionit 0,5 bis 2 mol Bicarbonat enthalten sowie soviel Dithionit/Bicarbonat-Gemisch, daß pro Mol Perfluorethyliodid 1 bis 5 mol Dithionit zum Einsatz gelangen.

3. Verfahren nach Anspruch 1, **dadurch gekennzeichnet, daß** man als Radikalinitiatoren komplexe Metallverbindungen in einer Menge von 1 bis 5 mol pro mol Perfluorethyliodid einsetzt.

4. Verfahren nach einem oder mehreren der Ansprüche 1 bis 3, **dadurch gekennzeichnet, daß** man Radikalinitiatoren in Mengen von 0,01 bis 0,5 mol pro Mol Pentafluorethyliodid einsetzt.

5. Verfahren Ansprüchen 1 bis 4, **dadurch gekennzeichnet, daß** man die Addition in Gegenwart eines Verdünnungsmittels durchführt.

6. Verfahren nach Ansprüchen 1 bis 5, **dadurch gekennzeichnet, daß** man die Addition mit Natriumdithionit/Natriumbicarbonat-Gemischen bei -50 bis +20°C durchführt.

7. Verfahren nach Ansprüchen 1 bis 6, **dadurch gekennzeichnet, daß** man Pentafluorethyliodid und Allylalkohol in molaren Verhältnissen von 1:0,7 bis 20 einsetzt.

8. Verfahren nach Ansprüchen 1 bis 7, **dadurch gekennzeichnet, daß** man als Katalysator Platinoxid, Palladium-auf-Kohle oder Raney-Nickel einsetzt.

9. Verfahren nach Ansprüchen 1 bis 8, **dadurch gekennzeichnet, daß** man pro Mol 4,4,5,5,5-Pentafluor-2-iod-1-pentanol 0,9 bis 2 Äquivalente Säurebindemittel und 100 bis 1 000 ml Verdünnungsmittel einsetzt.

## Claims

1. Process for preparing 4,4,5,5,5-pentafluoro-1-pentanol from perfluoroethyl iodide, **characterized in that** it comprises initially adding perfluoroethyl iodide in the presence of radical initiators selected from the groups consisting of dithionite/bicarbonate mixtures and complex metal compounds which contain one or more central atoms from the group consisting of iron, cobalt and nickel, and which contain dienyl and/or carbonyl ligands, to allyl alcohol and then hydrogenolytically dehalogenating the resulting 4,4,5,5,5-pentafluoro-2-iodo-1-pentanol in the presence of a catalyst, an acid binder which is selected from the group consisting of hydrogencarbonates, primary, secondary or tertiary amines or alkanolamines, and a diluent.

2. Process according to Claim 1, **characterized in that** dithionite/bicarbonate mixtures are employed which contain 0.5 to 2 mol of bicarbonate per mole of dithionite, and such an amount of dithionite/bicarbonate mixture is used that 1 to 5 mol of dithionite are employed per mole of perfluoroethyl iodide.

3. Process according to Claim 1, **characterized in that** the radical initiator used is a complex metal compound in an amount of from 1 to 5 mol per mole of perfluoroethyl iodide.

4. Process according to one or more of Claims 1 to 3, **characterized in that** radical initiators are employed in amounts of from 0.01 to 0.5 mol per mole of pentafluoroethyl iodide.

5. Process according to Claims 1 to 4, **characterized in that** the addition is carried out in the presence of a diluent.

6. Process according to Claims 1 to 5, **characterized in that** the addition is carried out using sodium dithionite/sodium bicarbonate mixtures at -50 to +20°C.

7. Process according to Claims 1 to 6, **characterized in that** pentafluoroethyl iodide and alkyl alcohol are employed in molar ratios of from 1:0.7 to 20.

8. Process according to Claims 1 to 7, **characterized in that** the catalyst used is platinum oxide, palladium on carbon or Raney nickel.

9. Process according Claims 1 to 8, **characterized in that** 0.9 to 2 equivalents of acid binder and 100 to 1000 ml of diluent are employed per mole of 4,4,5,5,5-pentafluoro-2-iodo-1-pentanol.

## Revendications

1. Procédé de préparation du 4,4,5,5,5-pentafluoro-1-pentanol à partir de l'iodure de perfluoroéthyle **caractérisé en ce que** l'on ajoute d'abord à de l'alcool allylique de l'iodure de perfluoroéthyle en présence d'initiateurs radicalaires choisis dans le groupe des mélanges dithionite/bicarbonate et des composés métalliques complexes qui contiennent un ou plusieurs atomes centraux du groupe du fer, du cobalt et du nickel et des ligands diényle et/ou carbonyle, après quoi on déshalogène par hydrogénolyse le 4,4,5,5,5-pentafluoro-2-iodo-1-pentanol ainsi obtenu en présence d'un catalyseur, d'un agent liant les acides qui est choisi dans le groupe des hydrogénocarbonates, des amines primaires, secondaires ou tertiaires et des alkanolamines, et d'un diluant.

2. Procédé selon la revendication 1 **caractérisé en ce que** l'on utilise des mélanges dithionite/bicarbonate qui contiennent 0,5 à 2 moles de bicarbonate par mol de dithionite ainsi qu'un mélange dithionite/bicarbonate en une quantité telle que 1 à 5 moles de dithionite sont utilisées par mole d'iodure de perfluoroéthyle.

3. Procédé selon la revendication 1 **caractérisé en ce que** l'on utilise comme initiateurs radicalaires des composés métalliques complexes en une quantité de 1 à 5 moles par mole d'iodure de perfluoroéthyle.

4. Procédé selon une ou plusieurs des revendications 1 à 3 **caractérisé en ce que** l'on utilise des initiateurs radicalaires en des quantités de 0,01 à 0,5 mole par mole d'iodure de pentafluoroéthyle.

5. Procédé selon les revendications 1 à 4 **caractérisé en ce que** l'on réalise l'addition en présence d'un diluant.

6. Procédé selon les revendications 1 à 5 **caractérisé en ce que** l'on réalise l'addition avec des mélanges dithionite de sodium/bicarbonate de sodium à - 50 à + 20°C.

7. Procédé selon les revendications 1 à 6 **caractérisé en ce que** l'on utilise l'iodure de pentafluoroéthyle et l'alcool allylique dans des proportions molaires de 1 : 0,7 à 20.

8. Procédé selon les revendications 1 à 7 **caractérisé en ce que** l'on utilise comme catalyseur de l'oxyde de platine, du palladium sur charbon ou du nickel de Raney.

9. Procédé selon les revendications 1 à 8 **caractérisé en ce que** l'on utilise par mole de 4,4,5,5,5-pentafluoro-2-iodo-1-pentanol, 0,9 à 2 équivalents d'agent liant les acides et 100 à 1 000 ml de diluant.
